# EUROPEAN PATENT APPLICATION

(11) **EP 1 927 342 A2**
(43) Date of publication of application: **04.06.2008**
(21) Application number: 07022473.8
(22) Date of filing: 20.11.2007
(51) Int. Cl.: A61K 8/31, A61K 8/34, A61K 8/39, A61K 8/67, A61Q 19/00

(54) **Hygienic and cosmetic compositions for treating sensitive skins**

(30) Priority: 21.11.2006 IT MI20062227
(71) Applicant: Betafarma S.p.A., 20090 Cesano Boscone (IT)
(72) Inventor: Tallia, Ettore, 20090 Cesano Boscone (MILANO) (IT)
(74) Representative: Cicogna, Franco

(57) **Abstract**

A hygienic and cosmetic compositions which, without a particular pharmacologic activity, are suitable for a functional differentiated and specific use on skins susceptible to cause symptoms related to sensitive, hyper-reactive dehydrated, dry hyper-keratosic skins typical of the senile age and confined to bed persons. The subject hygienic and cosmetic compositions being characterized in that the water included in their formulations is mostly bound with a low value of "free water", less than 0.7.

## Description

### BACKGROUND OF THE INVENTION

The present invention relates to hygienic and cosmetic compositions for treating or processing sensitive skins.

More specifically, the present invention relates to a series of hygienic and cosmetic products, which, without any particular pharmacologic activity, are suitable for a functional differentiated and specific use on skins generating symptoms related to sensitive, hyper-reactive, dehydrated, dry, hyper-keratosic skins, typical of the senile age and of the confined to bed persons.

In particular the application of the above products would be very useful on skins affected by chronic inflammatory symptoms, such as rosacea, or temporary symptoms, such as child napkin dermatitis.

The above mentioned skins are frequently affected by objectable burning or itching films, due to a clear and enhanced intolerance to particular components present, even in a small rates, in currently used cosmetic products (such as creams, lotions, milks), and which are made sensible by irritating outside environment agents, allergenic substances, intensive sun radiation, an uneven and insufficient hydrating of the hydro-lipidic surface mantle.

Current dermatologic knowledges suggest that sensitive and reactive skins would react, with a clear immune response, since their "Stratum Corneum", as a natural defensive barrier, is not sufficient to assure a proper physiologic protection.

The appearance of cutaneous hyper-reactivity and sensitivity phenomena depends, consequently, on a multi-factor concomitance of causes which cannot be easily detected and are actually not well definite.

A series of products for persons having sensitive skins, and which frequently must be subjected to extended and specific pharmacologic treatments, has been already provided.

The above prior products, having a comparatively high safety degree, are adapted to meet the most common and normal hygienic requirements, either alternately or in a complementary manner to the drug, with a beneficial preventive cosmetic additional effect.

Accordingly, the mentioned products are compositions having a skin-nourishing, emollient, protective, hydrating, cleansing, screening and so on property.

By properly formulating the above compositions, while properly choosing high purity components therefor, it is possible to achieve, in the composition, a nearly full exclusion of known components or other components suspected of having a sensitizing effect, or a skin-irritating, delidipifying, dehydrating or allergizing effect.

From the dermatologic literature, the following problems have been found, which have been also constantly confirmed by clinic workers.
A) The cosmetic preserving agents, even if they are admitted for use according to current legislative rules, frequently cause itching or irritating, skin sensibilization and allergizing phenomena, in a lot of persons.
   Actually, molecules designed for preserving cosmetic products, and in particular those which are mostly used even if at a very low dose, cannot be well tolerated by some reactive skins.
   A self-evident example of the above mentioned phenomena are, for example, the cosmetic product classes including Parabens (Nipagines), Benzoates, Quaternari ammonium salts, Chlorohydroxydiphenylethers, Isothiazolidinic compounds, Bromine derivates, Formole and derivative thereof.
B) In addition to the above products, also normal alkaline soaps, and a SLS-SLES foaming substances, because of their high delidipifying and dehydrating power, represent substance of a comparatively unsafe use in reactive skins.
C) Glycols, low boiling alcohols, solvents, dyes, and perfumes also exacerbate, very frequently the skin reactive response.
D) Substances derived from the plant kingdom (oil extracts or glycol extracts of plants, flowers, herbs) or animal kingdom (milk proteins or organ extracts), because of their frequent allergizing power, clearly enhance objectable symptoms of sensitive skins.

### SUMMARY OF THE INVENTION

In the light of the above disclosure, the Applicant, to provide functional and safe hygienic-cosmetic products, has provided unique formulations excluding all the above mentioned starting material classes.

As is known, cosmetic products in general are frequently easily subjected to a microbical contamination because of the presence, in their formulation, of water and biodegrabale organic substances.

A self-preserving of a cosmetic and hygienic product can be achieved only if it is fully anhydrous or as a free water value (the so-called water activity) less than 0.7.

In fact, the most part of bacteria cannot survive for free water values less than 0.9, and molds and yeasts stop to grow at values less than 0.8.

As found by the Applicant, the above physical-chemical parameter is the most important and critical factor in controlling the deterioration, from a microbic attack, of several classes of products in the food, pharmaceutic and cosmetic field.

The free water value, as it has been found, is reversely proportional to the amount of water intimately bound to the formulated product (independently from the concentration thereof contained in the product, and which can be hardly transferred.

Accordingly, the free water of a product or compound, and not the overall water contents present therein, will set the water lower limit available for a possible microbical growth.

A portion of the total water is moreover strongly bound to specific sites, such as hydroxy polysaccharide groups, protein carbonyl and aminic groups, or other strong polar sites.

According to the invention, all the series of products for processing sensitive skins developed by the Applicant, having very high self-preserving characteristics, are, accordingly, necessarily rich in polar polyalcohols (such as glycerin, sorbitol, xylitol, mannitol and polysaccharides.

In particular, the polyalcohols present in the subject formulations or products, in addition to provide a basic function to prevent the products to be microbically degraded, further represent, as it would be well known to one skilled in the art, very good moistening and hydrorecovering skin substances.

They hold a proper skin hydrating physiology level, by properly adjusting the so-called "perspiratio insensibilis", while preventing skin to be imbibed or saturated by a water excess, thereby greatly reducing or just eliminating objectable reactions of dry, scaling and hyperkeratosic skins.

In addition to the foregoing, the "Stratum Corneum", operating as a skin natural defensive barrier, is such as to provide a high functional and physiologic improvement, as a consequence of an application of cosmetic products including non transferable "free water".

Actually, the above cosmetic products are adapted to control and partially hold on the skin, its "perspiratio insensibilis", with a great advantage from a hydrating standpoint.

The above disclosed formulation examples have been herein shown only by way of an illustrative example, to better clarify particular embodiments of the invention.

It should be apparent to one skilled in the art that the following formulations are susceptible to several modifications and variations, all of which will come within the scope of the invention as herein disclosed and claims.

### Examples

| **Cleansing and bath oil for sensitive skins:** | | |
|---|---|---|
| Components | % w/w | Functions |
| P.O.E. aliphatic alcohols | 1-5 | Emulsifier |
| Aliphatic esters | 0.5- 30 | Emollient |
| Vitamin E acetate | 0.05-0.5 | Anti-radical |
| F.U. petrolatum oil | 65-95 | Protecting-hydrating |

| **Bath foam generating shampoo for sensitive skins:** | | |
|---|---|---|
| Components | % w/w | Functions |
| Polyalcohols | 55-80 | Moistening-hydrating |
| Amphoteric foaming | 5-15 | Cleansing agents |
| Salified carbomer | 0.2-3 | viscosity enhancing |
| P.O.E. sorbitan-esters | 0-5 | Emulsifier |
| Maltodextrins | 0-5 | Refreshing |
| Magnesium sulphate | 0.1-1 | Protective |
| Vitamin E acetate | 0.05-0.5 | Antiradical |
| Depurated water | q.s to 100 | |

| **Cream for dehydrated hyper-keratosic sensitive skins:** | | |
|---|---|---|
| Components | % w/w | Functions |
| F.U. petrolatum oil | 10-20 | Protective-hydrating |
| Aliphatic esters | 5-15 | Emollient |
| Silicone oil | 3-10 | Emollient protective |
| Waxy aliphatic alcohols | 2-5 | Gelifying |
| P.O.E. aliphatic alcohols | 2-5 | Emulsifier |
| Starch | 1-5 | Refreshing |
| vitamin E acetate | 0.05-0.5 | Antiradical |
| Polyalcohols | 20-50 | Moistening-hydrating |
| Carbomer | 0.1-0.7 | viscosity enhancing |
| Triethanolamine | 0.2- 1 | Salifying |
| Depurated water | q.s. to 100 | |

| **Cleansing milk for sensitive skins:** | | |
|---|---|---|
| Components | % w/w | Functions |
| F.U. petrolatum oil | 5-30 | Cleansing-Hydrating protective |
| W/O emulsifier | 1-4 | Emulsifier |
| Gelifying waxes | 0.5-5 | Rheologic modifying |
| Aliphatic esters | 5-20 | Emollient |
| Polyalcohols | 20-55 | Moistening-hydrating |
| Starch | 1-5 | Refreshing |
| Vitamin E acetate | 0.05-0.5 | Antiradical |
| Magnesium sulphate | 0.5-3 | Protective |
| Depurated water | q.s. to 100 | |

| **Anti-sun fluid cream for sensitive skins:** | | |
|---|---|---|
| Components | % w/w | Functions |
| F.U. petrolatum oil | 5-20 | Protecting-hydrating |
| W/O emulsifier | 1-4 | Emulsifier |
| Gelifying waxes | 0.5-5 | Rheologic modifying |
| Aliphatic esters | 5-20 | Emollient |
| Polyalcohols | 20-55 | Moistening-hydrating |
| UVA - UVB filters | 5-20 | Anti-sun protective |
| Bioxide titanium | 0.5-5 | Anti-sun screening |
| Starch | 1-5 | Refreshing |
| Vitamin E acetate | 0.05-0.5 | Antiradical |
| Magnesium sulphate | 0.5-3 | Protective |
| Depurated water | q.s. to 100 | |

| **Rosacea lenitive ointment:** | | |
|---|---|---|
| Components | % w/w | Functions |
| F.U. petrolatum oil | q.s. to 100 | Protecting-hydrating |
| Styrene copolymer | 2-5 | Stabilizing |
| Ozocerite | 1-3 | Gelifying |
| UVA - UVB filters | 1-10 | Anti-sun protective |
| Zinc oxide | 2-10 | Absorption-lenitive |
| Starch | 1-5 | Refreshing |
| Vitamin E acetate | 0.05-0.5 | Antiradical |

| **Napkin anti-redness ointment:** | | |
|---|---|---|
| Components | % w/w | Functions |
| F.U. petrolatum oil | 40-70 | Protecting-hydrating |
| Styrene copolymer | 2-5 | Stabilizing |
| Ozocerite | 1-3 | Gelifying |
| Zinc oxide | 2-20 | Absorption-lenitive |
| Starch | 1-5 | Refreshing |
| Vitamin E acetate | 0.05-0.5 | Antiradical |

It has been found that the invention fully achieves its intended aim and objects.

The invention has been herein disclosed with reference to preferred embodiments and formulations thereof and it should be apparent that the invention is susceptible to several modifications and variations, all of which will come within the scope of the thereof.

## Claims

1. Hygienic and cosmetic compositions for treating sensitive skins, **characterized in that** said compositions comprise formulating water mainly bound with a "free water" value less than 0.7.

2. A composition according to claim 1,
**characterized in that** said composition is a foaming bath-shampoo cleansing composition comprising two or more of the following components, in a by weight rate:
Polyalcohols 55-80%,
Amphoteric foaming agents 5-15%,
Salified carbomer 0.2-3%,
P.O.E. sorbintan esters 0-5%,
Maltodestrins 0-5%,
Magnesium sulphate 0.1-1%,
Vitamin E acetate 0.05-0.5%,
Depurated water q.s. to 100.

3. A composition according to claim 1,
**characterized in that** said composition is a cleansing bath oil comprising two or more of the following components, in a by weight rate:
P.O.E. aliphatic alcohols 1-5%,
Aliphatic esters 0.5-30%,
Vitamin E acetate 0.05-0.5%,
F.U. petrolatum oil 65-95%,

4. A composition according to claim 1,
**characterized in that** said composition is a cream for sensitive dehydrated hyper-keratosic skins, which comprises two or more of the following components, in a by weight rate:
F.U. petrolatum oil 10-20%,
Aliphatic esters 5-15%,
Silicone oil 3-10%,
Waxy aliphatic alcohols 2-5%,
P.O.E. aliphatic alcohols 2-5%,
Starch 1-5%,
Vitamin E acetate 0.05-0.5%,
Polyalcohols 20-50%,
Carbomer 0.1-0.7%
Trietanolamine 0.2-1%
Depurated water q.s. to 100.

5. A composition according to claim 1,
**characterized in that** said composition is a cleansing milk, comprising two or more of the following components, in a by weight rate:
F.U. petrolatum oil 5-30%,
W/O emulsifier 1-4%,
Gelifying waxes 0.5-5%,
Aliphatic esters 5-20%,
Polyalcohols 20-55%
Starch 1-5%,
Vitamin E acetate 0.05-0.5%,
Magnesium sulphate 0.5-3%
Depurated water q.s. to 100.

6. A composition according to claim 1,
**characterized in that** said composition is a fluid anti-sun cream, comprising two or more of the following components, in a by weight rate:
F.U. petrolatum oil 5-20%,
W/O emulsifiers 1-4%,
Gelifying Waxes 0.5-5%,
Aliphatic esters 5-20%,
Polyalcohols 20-55%,
UVA-UVB filters 5-20%,
Titanium bioxide 0.5-5%,
Starch 1-5%
Vitamin E acetate 0.05-0.5%,
Magnesium sulphate 0.5-3%,
Depurated water q.s. to 100.

7. A composition according to claim 1,
**characterized in that** said composition is a rosacea lenitive ointment, comprising two or more of the following components, in a by weight rate:
Styrene copolymer 2-5%,
Ozocerite 1-3%,
UVA-UVB filters 1-10%,
Zinc oxide 2-10%,
Starch 1-5%,
Vitamin E acetate 0.05-0.5%,
Petrolatum oil q.s. to 100.

8. A composition according to claim 1,
**characterized in that** said composition is a napkin anti-redness ointment, comprising two or more of the following components, in a by weight rate:
Styrene copolymer 2-5%,
Ozocerite 1-3%,
Zinc oxide 2-20%,
Starch 1-5%,
Vitamin E acetate 0.05-0.5%,
Petrolatum oil q.s. to 100.
